(19) Europäisches Patentamt
European Patent Office
Office européen des brevets

(11) Publication number: 0 234 464 B1

(12) # EUROPEAN PATENT SPECIFICATION

(45) Date of publication of the patent specification: 05.09.90

(51) Int. Cl.⁵: A61K 31/51 // (A61K31/51, 31:375, 31:195)

(21) Application number: 87102120.0

(22) Date of filing: 14.02.87

(54) **Composition and method for reducing acetaldehyde toxicity.**

(30) Priority: 18.02.86 JP 34494/86

(43) Date of publication of application: 02.09.87 Bulletin 87/36

(45) Publication of the grant of the patent: 05.09.90 Bulletin 90/36

(84) Designated Contracting States: BE CH DE FR GB IT LI LU NL SE

(56) References cited:
Unlisted drugs, vol.21,no. 1, January 1969, page 10; Chatham, N.J., US
Unlisted Drugs, vol. 21, no. 6, June 1969, page 87; Chatham, N.J., US
Unlisted drugs, vol. 24., no.6, June 1972, page 86; Chatham, N.J., US
Chemical abstracts, vo. 92, no. 21, May 26, 1980, page 155, ref.no. 175514x; Columbus, Ohio, US K.Koba et al.:"Reconsideration of the influence of medicines on ethanol metabolism." & KAGOSHIMA DAIGAKU IGAKU ZASSHI, 1979, 31(2), 461-74

(73) Proprietor: Takeda Chemical Industries, Ltd., 27, Doshomachi 2-chome Higashi-ku, Osaka-shi Osaka-fu(JP)

(72) Inventor: Matsuoka, Masayoshi, 2-9, Habikigaoka 8-chome, Habikino Osaka 583(JP)
Inventor: Kito, Go, 23-201, 8 Honmachi 5-chome, Yao Osaka 581(JP)

(74) Representative: von Kreisler, Alek, Dipl.-Chem. et al, Deichmannhaus am Hauptbahnhof, D-5000 Köln 1(DE)

EP 0 234 464 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

The present invention relates to a composition and method for reducing acetaldehyde toxicity, especially for preventing and relieving katzenjammer.

Katzenjammer or hangover which occurs on drinking, particularly on excessive drinking, is characerized by various manifestations such as skin flushes, hot sensation, chest distress, headache, dull headache, nausea, vomiting, breath odor, urinous odor, and so on, and at times presents with cerebral edema, functional neuritis and other symptoms.

Currently, against such symptoms as heaviness in the stomach, nausea, heart-burn, various gastrointestinal remedies or crude drugs are generally ingested in the hope of relieving the uncomfortable symptoms.

It is generally acknowledged that a hangover is mainly caused by unmetabolized residues of acetaldehyde, which is a metabolic intermediate of alcohol, in the drinker's body. Therefore, it has been thought that reducing the blood level of acetaldehyde should help prevent and treat hangover symptoms and further contribute to the prevention and treatment of liver damages associated with acetaldehyde.

Herbert Sprince et al. studied the antagonistic effects of various drugs against the anesthetic and lethal effects of acetaldehyde in animals and reported that a combination of L-ascorbic acid, L-cysteine, and thiamine hydrochloride exhibits an excellent antagonizing effect against acetaldehyde toxicity. [Agents and Actions, Vol. 5/2, pp. 164-173 (1975)]. Today, in view of the high frequency of drinking in daily life and the increasing consumption of alcohol, a need has been keenly felt for the development of an etiotropically effective medication for the prevention and treatment of hangover symptoms apparently associated with the toxic action of acetaldehyde.

The present inventors found surprisingly that the addition of fursultiamine (TTFD), which is in common use as the so-called activated vitamin $B_1$, to a basal mixture of L-cysteine and L-ascorbic acid results in a remarkably greater acetaldehyde-antagonizing effect as compared with the conventional ternary mixture of L-cysteine, L-ascorbic acid and thiamine hydrochloride.

It was also found that the formulation containing activated vitamin $B_1$ increases mitocondrial acetaldehyde dehydrogenase activity in rats. Further, the present inventors found that the addition of ursodesoxycholic acid, a cholagogue, to the above formula of L-cysteine, L-ascorbic acid and fursultiamine results in a further potentiated antagonism against the anesthetic and lethal effects of acetaldehyde.

The present invention is the result of further investigations based on the above findings.

The present invention is therefore directed to a composition for reducing acetaldehyde toxicity comprising, as combined active components, an effective amount of

a) a compound of the formula:

$$\begin{array}{l} R\text{-}NH\text{-}CH\text{-}COOH \\ \qquad\quad\ | \\ \qquad\quad (CH_2)_n\text{-}R' \end{array} \qquad (I)$$

wherein R is hydrogen or an acyl group; R′ is thiol or sulfonic (sulfo) group; n is an integer of 1 to 2,

b) ascorbic acid or a salt thereof. The invention is also directed to the use of said composition for the production of a medicine for reducing acetaldehyde toxicity.

Referring to the above general formula (I), the acyl group denoted by R is exemplified by lower alkyl $(C)_{1\text{-}4}$ carbonyl groups such as acetyl, propionyl and so on. In the present invention, the L-form compound (I) is preferably employed but the racemic compound may also be used. Examples of (I) include L-cysteine, N-acetyl-L-cysteine, L-homocysteine, L-cysteic acid and L-homocysteic acid and the corresponding racemates. Moreover, the compound (I) may be a mineral acid salt such as L-cysteine hydrochloride or an alkali metal salt such as sodium L-cysteinate. Preferred is L-cysteine.

The ascorbic acid mentioned above may be L-ascorbic acid. The salt of ascorbic acid includes such physiologically acceptable salts as the sodium salt, calcium salt and so on.

The disulfide type thiamine derivative may be any of the known active vitamin $B_1$ compounds having the S-S linkage in the molecule. For example, the following compounds may be mentioned.

(i) Thiamine disulfide and its derivatives, such as thiamine disulfide (TDS), bisbentiamine (BTDS) bisbutitiamine (Bu-TDS), bisibutiamine and so on.

(ii) Thiamine alkyl disulfide derivatives, such as prosultiamine (TPD), fursultiamine (TTFD) and octotiamine (TATD).

In the present invention, said thiamine compound may be used either in its free form or as a physiologically acceptable salt such as hydrochloride, nitrate and other mineral acid salts. It should be understood that since the thiamine compound may interact with said compound (I), formulation is made so as to avoid direct contact of the two compounds.

In a further aspect of the present invention, a cholagogue is added to the above 3-component formulation to provide a product having a still improved acetaldehyde-detoxicating effect.

The cholagogue is exemplified by ursodesoxycholic acid, dehydrocholic acid, osalmid(hydroxyphenyl salicylamide), phenylpropanol, anethole trithione, cyclobutyrol calcium, cyclobutyrol, hymecromone, trepibutone and chenodeoxycholic acid, but is not limited to those mentioned. Thus, any component having hepatic circulation increasing activity or liver function improving activity can be employed. In the practice of the present invention, the use of cholic acid derivatives having steroid nuclei, particularly ursodesoxycholic acid, is preferred.

The composition having acetaldehyde-detoxicating effect of the present invention is not limited to a composition consisting of the above-mentioned three components or four components. If necessary, various vitamins and the like, such as calcium pantothenate, nicotinamide, riboflavine and tocopherol acetate, may be added to the composition.

The composition according to the present invention can be orally administered to human subjects. As to dosage forms, tablets, granules, capsules and other optional forms can be provided. For the manufacture of such preparations, the established pharmaceutical procedures such as sugar coating, granulation can be employed. Thus, solid preparations may be produced using excipients such as lactose and starch, crystalline cellulose and potassium hydrogen phosphate, lubricants such as magnesium stearate and talc, and binders such as starch, polyvinylpyrrolidone, methylcellulose and hydroxypropylcellulose. In the case of a preparation containing the compound (I) and a pharmaceutically active substance which would interact therewith, such as a disulfide type thiamine derivative, a sugar-coated tablet containing one of the components in the plain tablet core and the other in the sugar coat may be provided. Alternative methods include the method which comprises granulating these components into independent granules and blending them, the method which comprises coating some of the granules and tableting them with the remaining granules, and the nucleation tableting method in which the two components are formed into the core and the outer layer, respectively.

The dosage of each component may be selected generally from the following ranges.

(a) Compound (I)
150 to 300 mg/day
(b) Ascorbic acid or a salt thereof (as free ascorbic acid)
250 to 2000 mg/day
(c) A disulfide type thiamine derivative or a salt thereof (as free compound)
20 to 100 mg/day
(d) A cholagogue
20 to 150 mg/day

The above daily dose is administered before and/or after drinking, or preferably before and after drinking in two divided doses.

The composition according to the present invention has activity to effectively lower the blood concentration of acetaldehyde. Therefore, it is of considerable value as a therapeutic and prophylactic composition for hangover associated with drinking or further as an acetaldehyde-detoxicating agent. The composition has remarkably low acute toxicity ($LD_{50}$ orally in rats: >5000mg/kg).

Experimental Example I

Rats (body weights 250-280 g) fasted for I8 hours were pereviously dosed orally with each of test compositions Formula A to Formula G as shown in Table I. Control group received a corresponding volume of saline by the same route. After a predetermined time (45-60 min ), acetaldehyde (I370 mg/kg) was orally administered to the rats and the effect of Formula A to Formula G on the anesthetic and lethal effects of acetaldehyde was observed.

When I370 mg/kg of acetaldehyde was administered without prior treatment with the test compositions, the rats (control group) fell into anesthesia within several minutes,and then developed dyspnea and the like and 90% of them died within I to 6 hours.

| Formula | Components | Dosage (mg/kg) |
|---|---|---|
| A | Fursultiamine hydrochloride | 100 |
| | L-Ascorbic acid | 352 |
| | L-Cysteine | 169 |
| B | Thiamine hydrochloride | 100 |
| | L-Ascorbic acid | 352 |
| | L-Cysteine | 169 |
| C | L-Ascorbic acid | 352 |
| | L-Cysteine | 169 |
| D | Fursultiamine hydrochloride | 50 |
| | L-Ascorbic acid | 352 |
| | L-Cysteine | 169 |
| E | Fursultiamine hydrochloride | 20 |
| | L-Ascorbic acid | 352 |
| | L-Cysteine | 169 |
| F | Fursultiamine hydrochloride | 50 |
| | Ursodesoxycholic acid | 30 |
| | L-Ascorbic acid | 352 |
| | L-Cysteine | 169 |
| G | Fursultiamine hydrochloride | 20 |
| | Ursodesoxycholic acid | 30 |
| | L-Ascorbic acid | 352 |
| | L-Cysteine | 169 |

Note: Formulas A and C are a formulation for comparison.

(1) The results with Formula A, B and C are shown in Table 1.

Table 1: Antagonistic Effects of Formula A, B and C on the anesthetic and lethal effects of acetaldehyde

| Formula | Anesthesia (%) | Mortality (%) | |
|---|---|---|---|
| | | After 1 hr | After 6 hr |
| Control | 81.2 (69/85) | 89.4 (76/85) | 90.6 (77/85) |
| A | 35.2 (19/54) | 33.3 (18/54) | 35.2 (19/54) |
| B | 60.0 (21/35) | 62.9 (22/35) | 71.4 (25/35) |
| C | 74.3 (26/35) | 60.0 (21/35) | 74.3 (26/35) |

( ): responsive cases/cases used

It will be apparent from Table I that whereas Formula C consisting of L-cysteine and L-ascorbic acid and Formula B consisting of Formula C plus thiamine hydrochloride are equivalent in effect, with the mortality due to acetaldehyde being approximately 70%, Formula A consisting of C plus fursultiamine showed a significantly superior antagonizing effect with a mortality of 35%.

The above results indicate that fursultiamine hydrochloride is more effective than thiamine hydrochloride.

(2) The results with Formulas D and E are shown in Table 2.

Table 2: Antagonistic Effects of Formula D and E on the anesthetic and lethal effects of acetaldehyde

| Formula | Anesthesia (%) | Mortality (%) | |
|---|---|---|---|
| | | After 1 hr | After 6 hr |
| Control | 83.2 (79/85) | 90.5 (86/95) | 91.5 (87/95) |
| D | 40.0 ( 8/20) | 30.0 ( 6/20) | 35.0 ( 7/20) |
| E | 60.0 (12/20) | 45.0 ( 9/20) | 45.0 ( 9/20) |
| ( ): | responsive cases/cases used | | |

In view of the confirmed effectiveness of fursultiamine hydrochloride in the above investigation (1), a dosage-finding study was conducted. It will be apparent from Table 2 that Formula D containing 50 mg/kg was as effective as Formula A containing 100 mg/kg and that even Formula E containing only 20 mg/kg showed a satisfactory result with a mortality of 45%. It is, therefore, considered that the use of fursultiamine hydrochloride is fully effective at the level of addition of 20 mg/kg.

(3) The results with Formulas F and G are shown in Table 3.

Table 3: Antagonistic Effects of Formula F and G on the anesthetic and lethal effects of acetaldehyde

| Formula | Anesthesia (%) | Mortality (%) | |
|---|---|---|---|
| | | After 1 hr | After 6 hr |
| Control | 84.8 (89/105) | 90.4 (95/105) | 91.4 (96/105) |
| F | 32.0 ( 8/ 25) | 30.0 ( 7/ 25) | 28.0 ( 7/ 25) |
| G | 32.0 ( 8/ 25) | 32.0 ( 8/ 25) | 40.0 (10/ 25) |
| ( ): | responsive cases/cases used | | |

The effect of addition of the cholagogue ursodesoxycholic acid to Formulas D and E was investigated. It will be apparent from Table 3 that Formulas F and G each supplemented with 30 mg/kg of ursodesoxycholic acid showed results more favorable than Formulas D and E.

(4) As a conclusion, a ternary composition of L-cysteine, L-ascorbic acid and fursultiamine hydrochloride antagonized the anesthetic and lethal effects of acetaldehyde. It was further found that the addition of ursodesoxycholic acid, having an increase in hepatic blood flow and an improvement in liver function, to the above ternary composition results in a further potent antagonistic effect.

The fact that the excellent antagonistic action of the present drug on the anesthetic and lethal effects of acetaldehyde was thus demonstrated in animal experiments suggest the likelihood that the drug is also effective in the prevention and treatment of hangover symptcms in man wherein acetaldehyde is primarily involved.

Experimental Example II

The same experiment as Experimental Example I was carried out with each of test compositions Formula H and I and the results obtained are shown in Table 4.

| Formula | Components | Dosage (mg/kg) |
|---|---|---|
| H | bisibutiamine | 50 |
| | L-ascorbic acid | 352 |
| | L-cysteine | 169 |
| I | bisibutiamine (BTDS) | 50 |
| | L-ascorbic acid | 352 |
| | L-cysteine | 169 |

Table 4: Antagonistic Effects of Formula H and I on the anesthetic and lethal effects of acetaldehyde

| Formula | Anesthesia (%) | Mortality (%) | |
|---|---|---|---|
| | | After 1 hr | After 6 hr |
| Control | 100 (30/30) | 100 (30/30) | 100 (30/30) |
| H | 40.0 (12/30) | 33.3 (10/30) | 46.7 (14/30) |
| I | 43.3 (13/30) | 43.3 (13/30) | 50.0 (15/30) |
| ( ): | responsive cases/cases used | | |

Experimental Example III

Sugar-coated tablets prepared according to the under-mentioned formula were administered orally to human subjects for a clinicopharmacological study (hereinafter referred to briefly as the drinking test) using the blood ethanol and acetaldehyde concentrations and the time course of hangover symptoms after alcohol loading as indicators.

| Formula | Amount |
|---|---|
| L-Cysteine | 240 mg |
| L-Ascorbic acid | 500 mg |
| Fursultiamine | 25 mg |
| Ursodesoxycholic | 30 mg |
| | (in 6 tablets) |

(1) Healthy volunteers abstained from drinking for 24 hours prior to the study were instructed to drink 2 g/kg of alcohol (wisky diluted with carbonated water) in about an hour and the subsequent course of blood ethanol and acetaldehyde concentrations was investigated. The time course of hangover symptoms (hot flushes, heat sensation, chest distress, headache, dull headache and nausea) was also monitored.

For an objective assessment of effects, the study was conducted in a single blind cross-over design using the active drug and its placebo.

The drug was administered (3 tablets per dose) in 2 doses, one hour before initiation of drinking and two hours after initiation of drinking. The results are given in Table 5.

It will be apparent from Table 5 that whereas no difference was found between active drug and placebo in blood ethanol concentration, the blood acetaldehyde level showed an overt decrease with the active drug as compared to the placebo. In correspondence with the decrease in blood acetaldehyde concentration, such hangover symptoms as hot flushes, heat sensation, chest distress, headache, dull headache, nausia, breath odor based probably on aldol component and urinous odor were also abated.

Table 5: Drinking test

| Subject | | 52-year-old male (M.M) | | 48-year-old male (F.N) | |
|---|---|---|---|---|---|
| Time after initiation of drinking (min.) | Parameter | $CH_3CHO$ (µM/l) | $C_2H_5OH$ (mM/l) | $CH_3CHO$ (µM/l) | $C_2H_5OH$ (mM/l) |
| 60 | | 4.28 | 43.82 | 4.80 | 36.82 |
| 240 | Placebo | 4.35 | 32.82 | 4.09 | 18.88 |
| 300 | | 3.13 | 31.60 | 4.03 | 17.91 |
| 60 | | 2.75 | 46.77 | 2.62 | 39.20 |
| 240 | Active drug | 3.07 | 32.60 | 1.79 | 29.08 |
| 300 | | 2.88 | 29.52 | 2.68 | 29.14 |

Table 5: Drinking test (continued)

| Subject | | 35-year-old male (K.Y) | | 24-year-old male (T.Y) | |
|---|---|---|---|---|---|
| Time after initiation of drinking (min.) | Parameter | $CH_3CHO$ (µM/l) | $C_2H_5OH$ (mM/l) | $CH_3CHO$ (µM/l) | $C_2H_5OH$ (mM/l) |
| 60 | | 7.91 | 34.60 | 5.95 | 22.28 |
| 240 | Placebo | 4.53 | 24.76 | 5.17 | 23.52 |
| 300 | | 4.66 | 22.40 | 6.81 | 23.31 |
| 60 | | 4.72 | 33.50 | 3.36 | 12.52 |
| 240 | Active drug | 3.89 | 23.60 | 4.68 | 20.55 |
| 300 | | 3.95 | 19.08 | 5.86 | 15.10 |

Table 5: Drinking test (continued)

| Subject | | 24-year-old male (H.T) | | 30-year-old male (T.F) | |
|---|---|---|---|---|---|
| Time after initiation of drinking (min.) | Parameter | $CH_3CHO$ (µM/l) | $C_2H_5OH$ (mM/l) | $CH_3CHO$ (µM/l) | $C_2H_5OH$ (mM/l) |
| 60 | | 4.31 | 23.60 | 16.17 | 18.68 |
| 240 | Placebo | 3.76 | 28.51 | 36.22 | 27.71 |
| 300 | | 3.45 | 19.57 | 19.19 | 23.61 |
| 60 | | 3.36 | 15.22 | 12.02 | 14.70 |
| 240 | Active drug | 2.80 | 21.56 | 7.99 | 52.95 |
| 300 | | 6.14 | 35.57 | 7.32 | 33.75 |

Table 5: Drinking test (continued)

| Subject | | 35-year-old male (T.A) | |
|---|---|---|---|
| Time after initiation of drinking (min.) | Parameter | $CH_3CHO$ (µM/l) | $C_2H_5OH$ (mM/l) |
| 60 | | 33.42 | 24.75 |
| 240 | Placebo | 8.30 | 32.65 |
| 300 | | 21.43 | 36.77 |
| 60 | | 20.93 | 38.47 |
| 240 | Active drug | 7.60 | 37.52 |
| 300 | | 9.48 | 42.51 |

The above Experimental Examples I, II and III revealed the following. The formulation consisting of L-cysteine, L-ascorbic acid, fursultiamine hydrochloride and ursodesoxycholic acid has proved to have detoxicating effects against the toxicity of acetaldehyde which is said to be a primary cause of hangover symptoms in animal experiments. Further, in the clinicopharmacological study in healthy volunteers, the above formulation promoted clearance of blood acetaldehyde to thereby display prophylactic and therapeutic effects against hangover symptoms.

<u>Example 1</u>

By means of a compression tableting machine, plain tablets were first prepared using the following ingredients in the following amounts per 6 tablets.

Ascorbic acid 500 mg
L-Cysteine 240 mg
Starch 280mg
Lactose 500 mg
Magnesium stearate 10 mg

Then, in a coating pan, the plain tablets were sugar-coated with a syrup and a spray composition containing 25 mg (per 6 tablets; the same applies hereinafter) of fursultiamine hydrochloride to give sugar-coated tablets.

Example 2

By means of a compression tableting machine, plain tablets were first prepared using the following ingredients in the following amounts per 6 tablets. Then, in a coating pan, the plain tablets were sugar-coated with a syrup and a spray composition containing 50 mg of fursultiamine hydrochloride to give sugar-coated tablets.
Ascorbic acid 500 mg
L-Cysteine 240 mg
Starch 250 mg
Lactose 500 mg
Magnesium stearate 10 mg
Then, in a coating pan, the plain tablets were sugar-caoated with a syrup and a spray composition containing 50mg of fursultiamine hydrochloride to give sugar-coated tablets.

Example 3

By means of a compression tableting machine, plain tablets were prepared using the following ingredients in the following amounts per 6 tablets.
Ascorbic acid 250 mg
L-Cysteine 240 mg
Starch 530 mg
Lactose 500 mg
Magnesium stearate 10 mg
In a coating pan, the above plain tablets were coated with a syrup and a spray composition containing 25 mg of fursultiamine hydrochloride to give sugar-coated tablets.

Example 4

By means of a compression tableting machine, plain tablets were first prepared using the following ingredients in the following amounts per 6 tablets.
Ascorbic acid 500 mg
L-Cysteine 240 mg
Ursodesoxycholic acid30 mg
Starch 250 mg
Lactose 500 mg
Magnesium stearate 10 mg
In a coating pan, these plain tablets were coated with a syrup and a spray composition containing 25 mg of fursultiamine hydrochloride to give sugar-coated tablets.

Example 5

By means of a compression tableting machine, plain tablets were first prepared using the following ingredients in the following amounts per 6 tablets.
Ascorbic acid 500 mg
L-Cysteine 240 mg
Ursodesoxycholic acid 30 mg
Starch 240 mg
Lactose 500 mg
Magnesium stearate 10 mg
In a coating pan, these plain tablets were coated with a syrup and a spray composition containing 34.3 mg of fursultiamine hydrochloride to give sugar-coated tablets.

## Claims

1. A composition for reducing acetaldehyde toxicity, which comprises, as combined active components, an effective amount of
a) a compound of the formula:

$$\begin{array}{l} R\text{-}NH\text{-}CH\text{-}COOH \\ \qquad\qquad | \\ \qquad\quad (CH_2)_n\text{-}R' \end{array}$$

wherein R is hydrogen or an acyl group; R′ is thiol or sulfonic group; n is an integer of 1 or 2,
b) ascorbic acid or a salt thereof and
c) a disulfide type thiamine derivative or a salt thereof.

2. The composition of Claim 1, wherein a cholagogue
d) is added to the 3-component formulation.

3. The composition of Claim 1, wherein the component
a) is in an amount of 150 to 300 mg, the component
b) is in an amount of 250 to 2000 mg and the component
c) is in an amount of 20 to 100 mg as a daily dose.

4. The composition of Claim 2, wherein the component
a) is in an amount of 150 to 300 mg, the component
b) is in an amount of 250 to 2000 mg, the component
c) is in an amount of 20 to 100 mg and the component
d) is in an amount of 20 to 150 mg as a daily dose.

5. The composition of Claims 3 and 4, wherein the daily dose is orally administered before and/or after drinking.

6. The composition of Claim 1, wherein the composition is a composition for preventing and relieving hangover symptoms.

7. Use of a composition comprising:
a) a compound of the formula:

$$\begin{array}{l} R\text{-}NH\text{-}CH\text{-}COOH \\ \qquad\qquad | \\ \qquad\quad (CH_2)_n\text{-}R' \end{array}$$

wherein R is hydrogen or an acyl group; R′ is thiol or sulfonic group; n is an integer of 1 or 2,
b) ascorbic acid or a salt thereof and
c) a disulfide type thiamine derivative or a salt thereof for the production of a medicine for reducing acetaldehyde toxicity.

## Patentansprüche

1. Zusammensetzung zur Reduzierung der Acetaldehyd-Toxizität, umfassend als kombinierte aktive Komponenten eine wirksame Menge
a) einer Verbindung der Formel

$$\begin{array}{l} R\text{-}NH\text{-}CH\text{-}COOH \\ \qquad\qquad | \\ \qquad\quad (CH_2)_n\text{-}R' \end{array}$$

in der R Wasserstoff oder eine Acyl-Gruppe ist, R′ Thiol oder eine Sulfon-Gruppe ist und n eine ganze Zahl 1 oder 2 ist,
b) Ascorbinsäure oder eines Salzes derselben und
c) eines Thiamin-Derivats des Disulfid-Typs oder eines Salz desselben.

2. Zusammensetzung nach Anspruch 1, worin der Drei-Komponenten-Formulierung ein Cholagogum
d) zugesetzt wird.

3. Zusammensetzung nach Anspruch 1, worin die Komponente
a) in einer Menge von 150 bis 300 mg, die Komponente
b) in einer Menge von 250 bis 2000 mg und die Komponente
c) in einer Menge von 20 bis 100 mg als Tagesdosis vorliegt.

4. Zusammensetzung nach Anspruch 2, worin die Komponente
a) in einer Menge von 150 bis 300 mg, die Komponente
b) in einer Menge von 250 bis 2000 mg, die Komponente
c) in einer Menge von 20 bis 100 mg und die Komponente
d) in einer Menge von 20 bis 150 mg als Tagesdosis vorliegt.

5. Zusammensetzung nach den Ansprüchen 3 und 4, worin die Tagesdosis oral vor und/oder nach dem Trinken verabreicht wird.

6. Zusammensetzung nach Anspruch 1, worin die Zusammensetzung eine Zusammensetzung zur Verhütung und Linderung der "Kater"-Symptome ist.

7.Verwendung einer Zusammensetzung, umfassend
a) eine Verbindung der Formel

$$R-NH-CH-COOH$$
$$(CH_2)_n-R'$$

in der R Wasserstoff oder eine Acyl-Gruppe ist, R' Thiol oder eine Sulfon-Gruppe ist und n eine ganze Zahl 1 oder 2 ist,
b) Ascorbinsäure oder ein Salz derselben und
c) ein Thiamin-Derivat des Disulfid-Typs oder ein Salz desselben
zur Herstellung eines Medikaments zur Reduzierung der Acetaldehyd-Toxizität.

**Revendications**

1. Composition pour réduire la toxicité de l'acétaldéhyde, qui comprend, en tant que composants acitifs combinés, une quantité efficace de
a) un composé de formule

$$R-NH-CH-COOH$$
$$(CH_2)_n-R'$$

dans laquelle R représente un atome d'hydrogène ou un groupe acyle, R' représente un groupe thiol ou sulfonique, et n est un nombre entier valant 1 ou 2,
b) de l'acide ascorbique ou l'un de ses sels, et
c) un dérivé de thiamine de type disulfure ou un sel d'un tel composé.

2. Composition selon la revendication 1, dans laquelle un cholagogue (d) est ajouté à la formulation à trois composants.

3. Composition selon la revendication 1, dans laquelle, pour une dose quotidienne, la quantité du composant (a) vaut 150 à 300 mg, la quantité du composant (b) vaut 250 à 2000 mg, et la quantité du composant (c) vaut 20 à 100 mg.

4. Composition selon la revendication 2, dans laquelle, pour une dose quotidienne, la quantité du composant (a) vaut 150 à 300 mg, la quantité du composant (b) vaut 250 à 2000 mg, la quantité du composant (c) vaut 20 à 100 mg, et la quantité du composant (d) vaut 20 à 150 mg.

5. Composition selon l'une des revendications 3 et 4, la dose quotidienne étant administrée par voie orale avant et/ou après boire.

6. Composition selon la revendication 1, qui est une composition destinée à la prévention et au soulagement des symptomes de la "gueule de bois".

7. Utilisation d'une composition comprenant:
a) un composé de formule

$$R-NH-CH-COOH$$
$$(CH_2)_n-R'$$

dans laquelle R représente un atome d'hydrogène ou un groupe acyle, R' représente un groupe thiol ou sulfonique, et n est un nombre entier valant 1 ou 2,
b) de l'acide ascorbique ou l'un de ses sels, et
c) un dérivé de thiamine du type disulfure ou un sel d'un tel composé,
pour la production d'un médicament destiné à réduire la toxicité de l'acétaldéhyde.